# EUROPEAN PATENT SPECIFICATION

(11) **EP 0 755 397 B1**
(45) Date of publication and mention of the grant of the patent: **19.08.1998**
(21) Application number: 95921797.7
(22) Date of filing: 31.05.1995
(51) Int. Cl.: C07D 475/04

(54) **A PROCESS FOR THE PREPARATION AND SEPARATION OF DIASTEREOMERIC SALTS OF FOLINIC ACID**
VERFAHREN ZUR HERSTELLUNG UND TRENNUNG VON DIASTEREOMEREN SALZEN VON FOLINSÄURE
PROCEDE DE PREPARATION ET DE SEPARATION DE SELS DIASTEREOISOMERES DE L'ACIDE FOLINIQUE

(30) Priority: 08.06.1994 IT MI941193
(43) Date of publication of application: 29.01.1997
(73) Proprietor: BRACCO S.p.A., 20134 Milano (IT); DIBRA S.p.A., I-20122 Milano (IT)
(72) Inventor: FELDER, Ernst, I-20134 Milano (IT); RIPA, Giorgio, I-20134 Milano (IT); DISTASO, Carlo, I-20134 Milano (IT)
(74) Representative: Minoja, Fabrizio
(86) International application number: EP9502073
(87) International publication number: WO9533749

(56) References cited:
- EP-A- 0 455 013
- WO-A-93/17022
- DE-A- 4 136 921

## Description

This invention refers to a process for the preparation, separation and purification of stereoisomers of the salts of folinic acid which, in its calcium salt form, is used, in antitumoral therapy, as an antidote for the folic acid antagonists, such as antagonists of aminopterine or methotrexate. As a matter of fact, these substances block the metabolism of folic acid in the organism, preventing the transformation of dihydrofolic acid into tetrahydrofolic acid. On the contrary, in combination with another antitumoral drug, i.e. 5-fluoroacyl, calcium folinate increases the activity of folic acid. The folinic acid calcium salt is also used in all anaemic forms deriving from the lack of folates.

The process of the invention allows the preparation of the two diastereomeric forms of said salts in a convenient way, in good yields and with an high optical purity.

Folinic acid, N-(5-formyl-(6RS)-5,6,7,8-tetrahydropteroyl)-L-glutamic acid, when synthetically obtained, consists of the equimolar mixture of two diastereomeric forms, (6R) and (6S) respectively.

It is known that only the (6S) isomer, as calcium salt, meets the desired pharmacological activity: therefore processes allowing the preparation of said optically pure (6S) form have been extensively studied.

Several attempts were made to obtain the (6S) isomer through asymmetric synthesis [Rees L., Valente E., Suckling C. J., Wood H.C.S. - Tetrahedron Vol. 42 - no. 1, pag. 117-136, 1986; Rees L., Suckling C. J., Wood H.C.S. - J. Chem. Soc., Chem. Commun., pag. 470-472, 1987; Brunner H., Huber C., Dublack P., EP551642-Al (BASF-AG)], but none turned out to be industrially successful. Therefore most of the R&D work focused on the separation of the (6RS) diastereomeric mixture obtained by known synthetic ways, such as for instance, the one described by C. Temple and Coll. [US 4,148,999; US 4,206,307; J. Med.Chem. 22, 731 (1979)].

An example of the state of the art concerning said separation of (6R) and (6S) diastereomers from their equimolar mixture is given by the following list: US 2,688,018; EP-A 0266042; EP-A 0348641; EP-A 0356934; EP-A 0367902; EP-A 0432441; EP-A 0455013; EP-A 0495204; WO 9113890; WO 8808844; WO 9317022.

The problem has not yet been solved as far as the industrial production is concerned, either related to the global yield, or to the optical purity of the obtained diastereomers.

This invention refers to a process which allows, starting from a known key intermediate, namely (6RS)-5,10-methenyl-5,6,7,8-tetrahydrofolic acid chloride hydrochloride [for instance obtained according to C. Temple; US 4,148,999; US 4,206,307; J. Med.Chem. 22, 731 (1979)], the preparation, separation and isolation of the diastereomeric salts of folinic acid with an at least dibasic organic amine, obtaining chemical and optical purity levels so high that the following transformation into the desired corresponding calcium salts is possible without performing further complex purification steps, providing therefore definite advantages from the industrial point of view.

As reported in Scheme 1, the folinic acid calcium salt is usually prepared starting from folic acid, which contains a (S) chiral centre in the part of the molecule corresponding to (S)-glutamic acid. By hydrogenation of the double bond between the 5- and 6-positions of the pterinic residue, a new chiral centre is formed in position 6 with a (6RS) configuration. The subsequent formylation of the nitrogen at the 5-position leads to the production of (6RS)-5,10-methenyl-5,6,7,8-tetrahydrofolic acid formate. By acidification with hydrochloric acid the crystallization of the corresponding chloride hydrochloride is obtained.

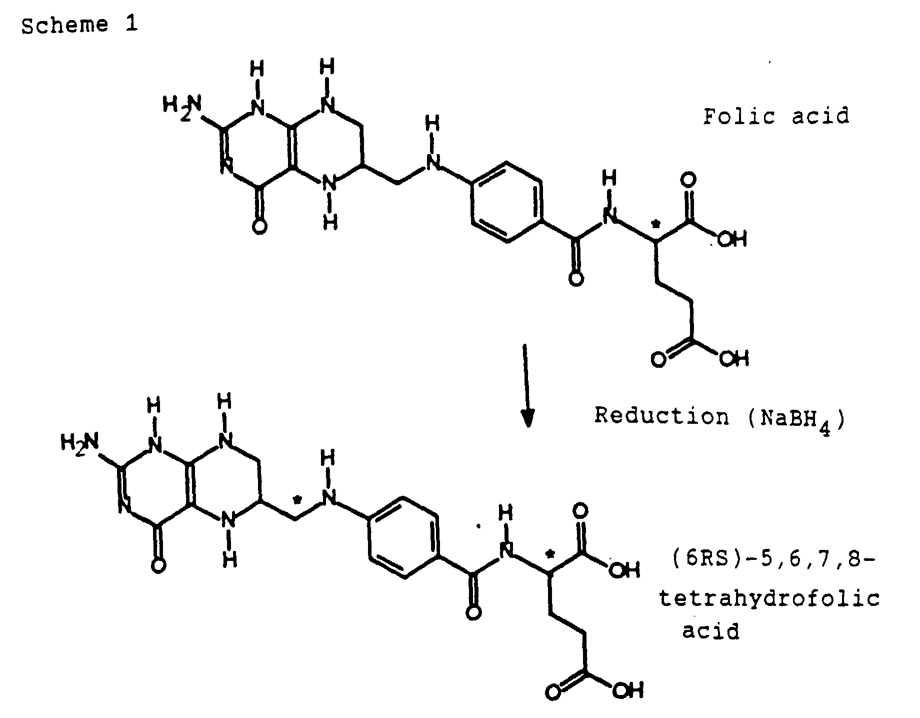

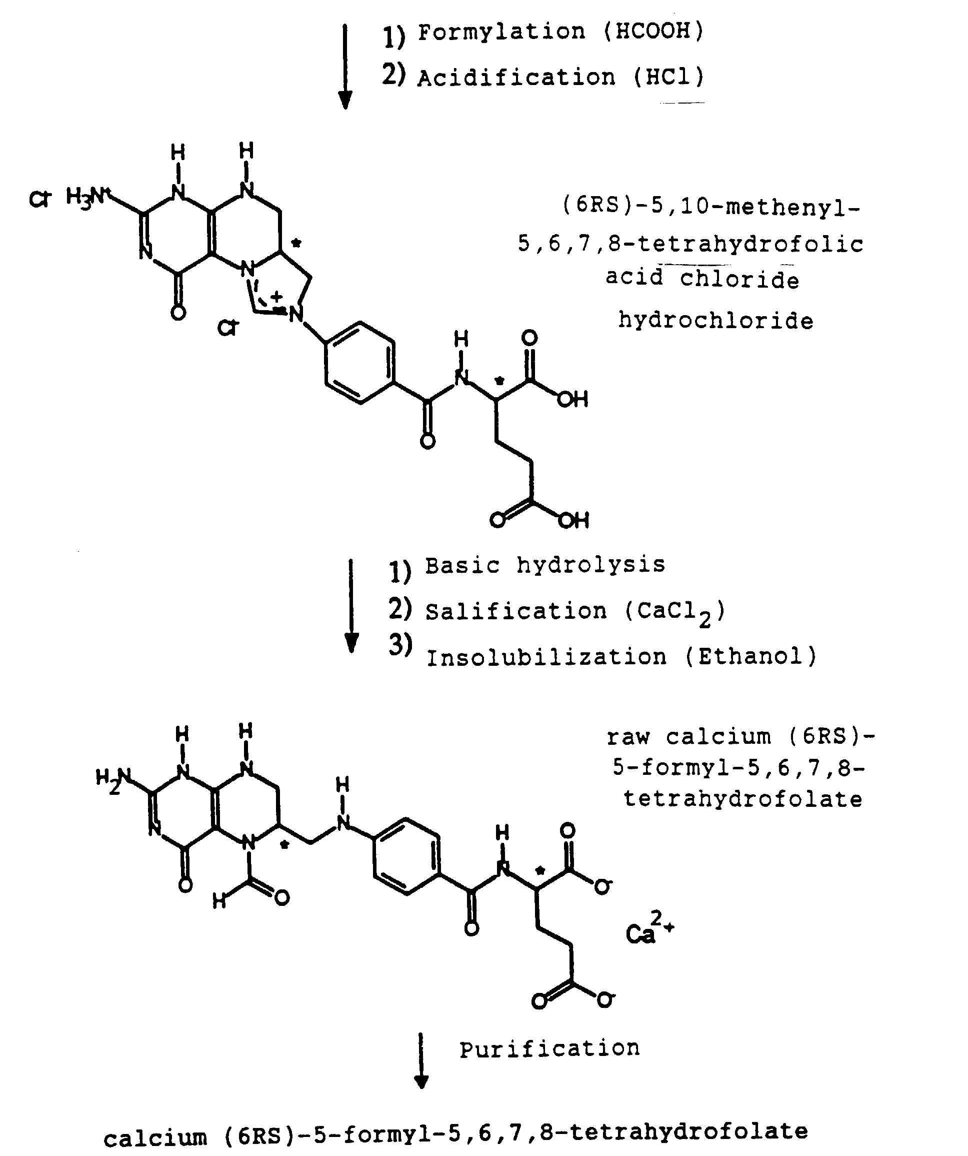

This intermediate is hydrolyzed, preferably with strong inorganic bases (for instance NaOH), or with strongly basic organic monoamines such as triethylamine, and under strictly controlled pH conditions.

The mixture of diastereomeric salts of folinic acid is transformed into the mixture of the corresponding calcium salts (with CaCl₂). Said mixture is precipitated from ethanol to give (6RS) crude calcium folinate which is purified through various purification steps. By treating with acids an aqueous solution of purified (6RS) calcium folinate, (6RS) folinic acid precipitates which is ready for the diastereomeric separation.

The series of above disclosed operations is quite complex, especially due to the fact that the specificity of the reaction of hydrolysis of (6RS)-5,10-methenyl-5,6,7,8-tetrahydrofolic acid chloride hydrochloride is not very high; therefore a large number of impurities are produced causing a decrease in the reaction yield, so that the purification of calcium folinate has to be performed before carrying out the isolation of folinic acid and the subsequent separation of the diastereomers.

Now it has surprisingly been found that, in contrast to the prior art methods, said hydrolysis in water or in a water/aprotic dipolar solvent mixture reaction can be performed in the presence of a suitable amount of a relatively weak base, consisting of an at least dibasic organic amine, which allows a high conversion and selectivity of the reaction. Moreover said diamine, in addition to its hydrolyzing function, acts at the same time as salifying agent for the folinic acid which has been formed.

As a consequence, the final crude solution, which contains the mixture of the diastereomeric diamine salts of (6RS)-folinic acid with diamine, can be directly used in order to selectively crystallize one of the two diastereomeric forms with an optical purity usually higher than 95%.

The type of diamine used highly affects the selectivity of the crystallization: for instance the (6S) diamine salt can be isolated first and then the corresponding (6R) form can be recovered from the crystallization mother liquors. For instance this occurs when piperazine is used as dibasic amine.

The opposed effect can equally be achieved. When ethylenediamine is used as a base, then the first isomer which crystallizes is the (6R) salt and the corresponding (6S) form can subsequently be recovered from mother liquors by a further crystallization.

The preferential crystallization of the less water-soluble diastereomeric salt from the crude solution of the hydrolysis reaction can be carried out according to known methods (WO 9317022, BRACCO). Preferably said solution is suitably diluted with solvents such as a dipolar aprotic solvent or an organic protic solvent or a mixture thereof, in order to promote the crystallization of the less water-soluble form.

Meanwhile, the removal of reaction impurities and side-products, remaining in solution, is obtained to such an extent that just one recrystallization of the diamine salt gives an optical purity higher than 99%.

In the most favourable cases, the optical and chemical purities of the diamino folinate diastereomer directly crystallized from the crude product deriving from hydrolysis are so high that they allow an easy conversion of said salt into the corresponding calcium salt without further purifications.

The stoichiometry of the hydrolysis reaction of (6RS)-5,10-methenyl-5,6,7,8-tetrahydrofolic acid chloride hydrochloride to diamine salt of folinic acid depends on the basicity of the amino groups of the used diamine.

For instance, ethylenediamine and 1,3-diamino-2-propanol are used in a 2:1 molar ratio to the starting product (i.e. in a stoichiometric ratio to the quantity of acid to be neutralized). For piperazine and N,N-dimethyl piperazine, less basic than the previous ones, a 3:1 molar ratio is preferred. Even in this case, the obtained diastereomeric salt consists of folinic acid and diamine in a 1:1 molar ratio, since each of the remaining two diamine moles neutralizes one equivalent of hydrochloric acid (for instance giving piperazine monohydrochloride).

Suitable at least dibasic organic amines may be selected from the group of aliphatic, linear, cyclic or heterocyclic, substituted or unsubstituted, racemic or optically active amines, containing at least two amino groups which are linked by at least a hydrocarbon chain, substituted or not, comprising at least 2 carbon atoms.

Particularly preferred amines are the diamines of the general formulae (I) to (III): 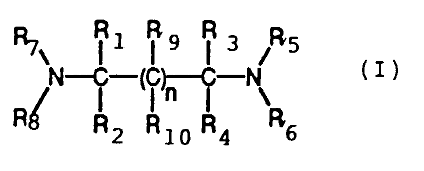 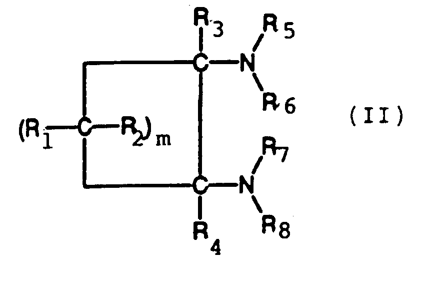 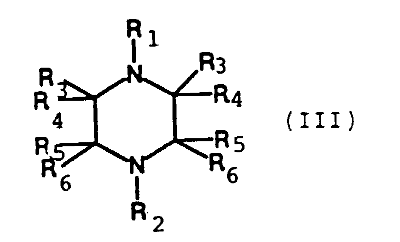 wherein:
- R₁→R₈: which are the same or different, are H or a linear or branched alkyl group, substituted or not by 1-4 OH groups;
- R₉→R₁₀: which are the same or different, have the same meanings as R₁-R₈ or represent hydroxy groups;
- n: is an integer from 0 to 6,
- m: is an integer from 2 to 8.

Examples of particularly preferred amines are selected from: ethylenediamine, 1,2-diamino-propane, 1,3-diamino-propane, 1,3-diamino-2-hydroxy-propane, (cis)-1,2-diamino-cyclohexane, (trans)-1,2-diaminocyclohexane, piperazine, 1,4-dimethyl-piperazine, 2-methyl-piperazine, 2,5-dimethylpiperazine.

Suitable solvents for the crystallization of diastereomeric mixtures of folinic acid salts with said amines are preferably binary or ternary mixtures of water/organic dipolar aprotic solvent or water/organic aprotic dipolar solvent/protic organic solvent.

Preferred aprotic dipolar solvents are, for example, dimethylformamide (DMF), dimethylacetamide (DMAC), dimethylsulfoxide (DMSO), N-methyl-pyrrolidone (NMP), hexamethylphosphoramide (HMPA).

Preferred organic polar protic solvents are methanol, ethanol, n-propanol, isopropanol, n-butanol, sec-butanol, ethylene glycol, 1,2-propylene glycol, 1,3-propylene glycol, formamide, N-methyl-formamide.

The process of this invention is described in the following Scheme 2 by using as starting products (6RS)-5,10-methenyl-5,6,7,8-tetrahydrofolic acid chloride hydrochloride, preferably prepared according to the procedure described by C. Temple [US 4,148,999; US 4,206,307; J. Med.Chem. 22, 731 (1979)], and, as an example of diamine, piperazine.

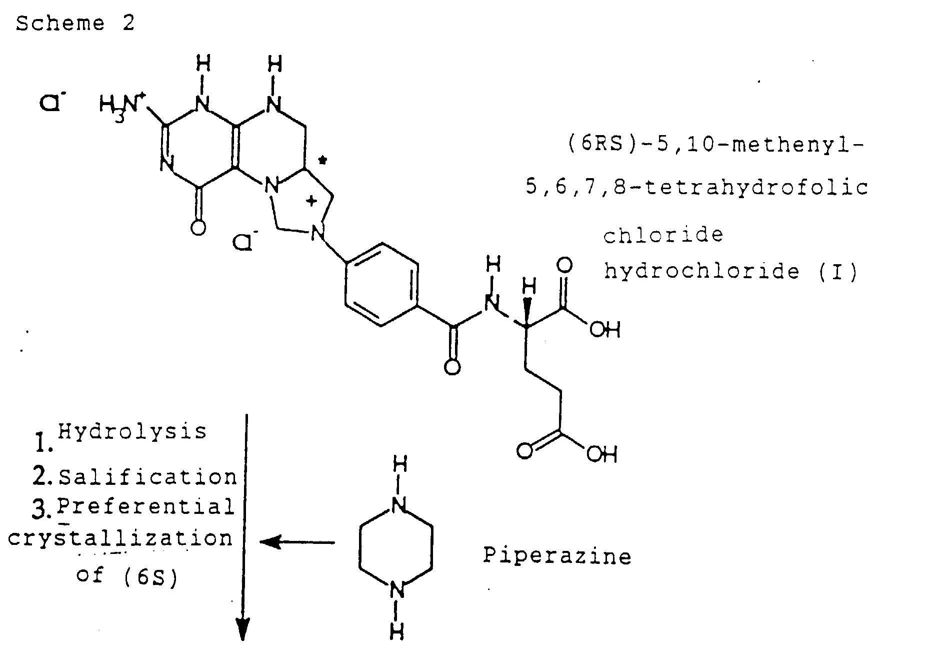

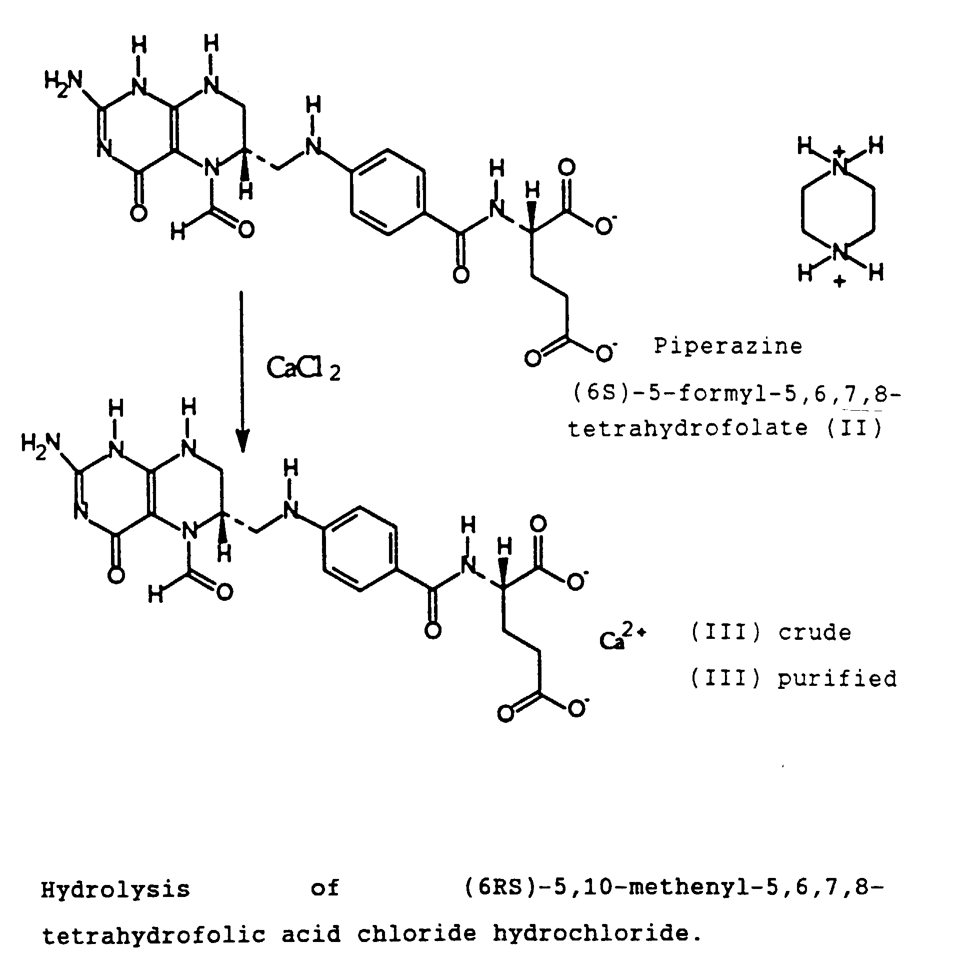

The reaction is preferably performed under the following conditions:
· acid/diamine molar ratio: from 1:2 to 1:4, preferably from 1:2 to 1:3.2
· solvent: water or a water/aprotic dipolar solvent mixture (for instance DMAC) in a weight ratio from 1:0.5 to 1:20
· acid/solvent mixture dilution ratio: from 1:2 to 1:80 w/w, preferably from 1:4 to 1:45 w/w
· temperature: from 60 to 100°C
· reaction time: from 3 to 12 h, preferably from 5 to 8 h.

The reaction is preferably performed under inert gas atmosphere.
Preferential crystallization of the less water-soluble diastereomer of diamine folinate from the crude solution after hydrolysis.

The crude solution obtained once the hydrolysis reaction is over, is cooled and then treated according to one of the following methods:
· possible further dilution with aprotic dipolar solvent (for instance DMAC) to increase the water/aprotic dipolar solvent ratio, preferably up to a maximum of 1:60 w/w.
· if necessary, further addition of an organic protic solvent (for instance EtOH), preferably in order to reach a maximum weight 12 times higher than the solution weight.

The crystallization is generally carried out at temperatures ranging from 0 to 25°C and for 10 to 120 h, preferably from 24 to 72 h.
Transformation of the diamine salt into the corresponding calcium salt.

The exchange takes place in an aqueous solution in the presence of an excess of calcium salt (preferably CaCl₂) at a nearly neutral pH, preferably under the following conditions:
· dilution ratio diamine salt/water: from 1:4 to 1:40 w/w
   preferably from 1:8 to 1:15 w/w
· diamine salt/CaCl₂ · 2H₂O ratio: from 1:1 to 1:6 w/w
   preferably from 1:3 to 1:5 w/w
· temperature: from 5 to 25°C
· crystallization time: from 10 to 60 h
· pH: between 6.5 and 7.5, if necessary with the addition of 1N NaOH.

From the residual mother liquors, if necessary after preferential crystallization of diamino folinate [respectively (6S) or (6R) according to the used diamine], even the other diastereomer can be recovered with an extremely high optical purity.

The preferred conditions are basically similar to those disclosed in WO 9317022 (BRACCO), and involve the crystallization of the diamine salt of the remaining diastereomer by dilution of mother liquors with a suitable solvent or solvent mixtures, its recrystallization and the subsequent transformation into the corresponding calcium salt.

Alternatively, the calcium salt can be obtained directly from the diamine salt in the starting mother liquors; recovery and purification can be carried out according to known methods (WO 9317022, BRACCO).

The following examples further illustrate the invention.

### Example 1

### Hydrolysis with piperazine and preferential crystallization of piperazine (6S)-folinate.

25 g of 5,10-methenyl-5,6,7,8-tetrahydrofolic acid chloride hydrochloride are suspended in 200 g of N,N-dimethylacetamide and in 49.0 g of water. The suspension is heated to 75°C, and 11.28 g of piperazine are added. The solution is heated to 80°C and is kept at this temperature for 5 h. It is then diluted with 250 g of N,N-dimethylacetamide and cooled to 15°C. Crystallization occurs at 15°C during 48 h. The crystallized solid is filtered, washed with 40 g of ethanol and dried. 9 g of piperazine (6S)-folinate with an optical purity higher than 98% are obtained.

### Example 2

### Conversion of piperazine (6S)-folinate into (6S)-calcium folinate.

8 g of piperazine (6S)-folinate, obtained according to example 1, are dissolved in 120 g of water. 32 g of calcium chloride dihydrate are added. pH is adjusted to 7 with 1N NaOH, then crystallization takes place at 16°C in 24 h. The crystallized solid is filtered, washed with aqueous ethanol and dried. 6 g of (6S)-calcium folinate, with an optical purity higher than 99% are obtained. The product is dissolved again in water and reprecipitated by addition of ethanol. 5.7 g of (6S)-calcium folinate, with an optical purity higher than 99%, free from chloride ions, are obtained.

### Example 3

### Isolation of crude piperazine (6R) folinate from crystallization mother liquors of example 1.

270 g of ethanol are added to the mother liquors of example 1. The solution crystallizes at 5°C in 64 h. The precipitated solid is then filtered off and washed with 40 g of ethanol. 8 g of piperazine (6R)-folinate with an optical purity higher than 90% are obtained.

### Example 4

### Preparation of piperazine (6R)-folinate with a high optical purity (>99%).

6 g of piperazine(6R)-folinate, obtained according to example 3, are dissolved at 50°C in 20 g of water and 110 g of N,N-dimethylacetamide. After cooling the solution to 25°C, 20 g of ethanol are added and the solution is cooled to 5°C. The product crystallizes in 64 h at 5°C. The crystals are filtered, washed with ethanol and dried. 5.0 g of piperazine (6R)-folinate with an optical purity higher than 99% are obtained.

### Example 5

### Conversion of piperazine (6R)-folinate into (6R)-calcium folinate.

4 g of piperazine (6R)-folinate, obtained according to example 4, are dissolved in 20 g of water. 12 g of calcium chloride dihydrate, dissolved into 20 g of water, are added. pH is adjusted to 7 with 1N NaOH. 100 g of ethanol are added to precipitate (6R)-calcium folinate. 3.2 g of (6R)-calcium folinate with an optical purity higher than 99% are obtained.

### Example 6

### Hydrolysis with ethylenediamine and preferential crystallization of crude ethylenediamine (6R)-folinate.

30 g of 5,10-methenyl-5,6,7,8-tetrahydrofolic acid chloride hydrochloride are suspended in 176 g of water and 204 g of N,N-dimethylacetamide (DMAC). The suspension is heated to 80°C and a solution of 6.3 g of ethylenediamine in 26.9 g of DMAC and 23.1 g of water is added dropwise in 3.5 h and kept under stirring at 80°C for 5 h. The solution is cooled at 5°C and left to stand for 24 h. The crystallized solid is filtered and washed with 15 g of ethanol, then dried. 10.3 g of ethylenediamine (6R)-folinate, 95.5% optically pure, are obtained.

### Example 7

### Isolation of ethylenediamine (6S)-folinate starting form mother liquors of example 6.

42.5 g of DMAC are added to the mother liquors of example 6. The solution is cooled to 10°C and seeded with ethylenediamine (6S)-folinate. After 92 h, the crystallized solid is filtered, washed with 10 g of ethanol, then dried. 2.6 g of ethylenediamine (6S)-folinate, 99% optically pure, are obtained.

### Example 8

### Preparation of (6S)-calcium folinate starting from the mother liquors of example 6.

80 g of calcium chloride dihydrate are added to the mother liquors of example 6. After addition of 900 g of ethanol, the solution is kept under stirring for 30 min, the precipitate is filtered, and washed with 40 g of ethanol. The product is dried, then redissolved in water and reprecipitated at pH 7 by addition of ethanol. 16 g of (6S)-calcium folinate, 75% optically pure, are obtained. The product is recrystallized three times from water, at pH 7 and in the presence of ^{~} 4 parts by weight of calcium chloride dihydrate. After dissolution in water and precipitation with ethanol, 5 g of (6S)-calcium folinate, with an optical purity higher than 99%, are obtained.

### Example 9

### Preparation of ethylenediamine (6R)-folinate with high optical purity (>99%).

5 g of ethylenediamine(6R)-folinate, obtained according to example 6, are recrystallized from 100 g of water/N,N-dimethylacetamide 1:1,16 w/w. 4 g of ethylenediamine (6R)-folinate with an optical purity higher than 99% are obtained.

### Example 10

### Preparation of (6R)-calcium folinate.

3.5 g of ethylenediamine(6R)-folinate, obtained according to example 9, are dissolved in 20 g of water. A solution of 12 g of calcium chloride dihydrate in 15 g of water is added. pH is adjusted to 7 with 1N NaOH, and 50 g of ethanol are added to precipitate (6R)-calcium folinate. 3 g of (6R)-calcium folinate with an optical purity higher than 99%, are obtained.

### Example 11

### Hydrolysis with 1,3-diamino-2-propanol and preferential crystallization of 1,3-diamino-2-propanol (6R)-folinate.

30 g of 5,10-methenyl-5,6,7,8-tetrahydrofolic acid chloride hydrochloride are suspended in 100 g of water and 220 g of N,N-dimethylacetamide (DMAC). The suspension is heated to 80°C then a solution of 9.5 g of 1,3-diamino-2-propanol in 80 g of water is added dropwise in 3.5 h. When the addition is over the solution is left at 80°C for 5 h. After cooling to 5°C, crystallization occurs in 48 h. The crystallized product is filtered, washed with 15 g of ethanol, then dried. 9.5 g of 1,3-diamino-2-propanol (6R)-folinate with a 95% optical purity are obtained.

### Example 12

### Preparation of 1,3-diamino-2-propanol (6S)-folinate.

45 g of DMAC are added to the mother liquors of example 11. The solution is cooled to 5°C and kept under stirring for 2 days, at 5°C. The crystallized product is filtered, washed with 5 g of ethanol, and dried. 2.5 g of 1,3-diamino-2-propanol (6S)-folinate with an optical purity higher than 98%, are obtained.

## Claims

1. A process for the preparation, separation and purification of (6S) and (6R) diastereomers of folinic acid salts comprising the hydrolysis of (6RS)-5,10-methenyl-5,6,7,8-tetrahydrofolic acid chloride hydrochloride and the separation of the 6R and 6S isomers characterized in that the hydrolysis is carried out with an at least dibasic amine in water or in a water/aprotic dipolar solvent mixture and that the obtained diastereoisomeric salts of (6RS) folinic acid with said at least dibasic amine are separated by selective crystallization directly from the crude solution obtained by the hydrolysis step, without previous purification, optionally followed by the conversion to calcium salts.

2. A process according to claim 1 wherein the at least dibasic amine is selected from the diamines of general formulae (I) to (III) 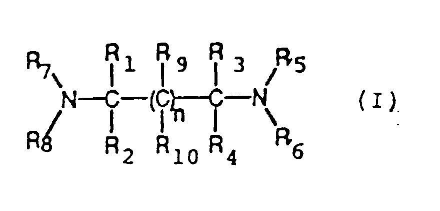 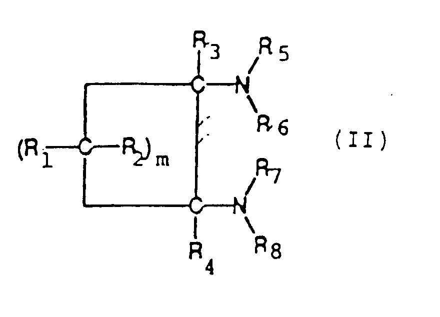 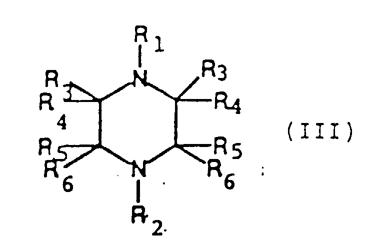 wherein:
R₁→R₈ which are the same or different, are H or a linear or branched alkyl group, substituted or not by 1-4 OH groups;
R₉→R₁₀ which are the same or different, have the same meanings of R₁-R₈ or represent hydroxy groups;
n is an integer from 0 to 6,
m is an integer from 2 to 8.

3. A process according to claim 2 wherein the preferred amine is selected from: ethylenediamine, 1,2-diamino-propane, 1,3-diamino-propane, 1,3-diamino-2-hydroxy-propane, (cis)-1,2-diamino-cyclohexane, (trans)-1,2-diamino-cyclohexane, piperazine, 2-methyl-piperazine, 1,4-dimethyl-piperazine, 2,5-dimethyl piperazine.

4. A process according to claim 1, wherein the dipolar aprotic solvent is selected from: dimethylformamide, dimethylacetamide, dimethylsulphoxide, N-methyl-pyrrolidone, hexamethylfosphoramide.

5. A process according to any one of claims 1-4 wherein the precipitation of the less soluble diastereomer of said diamino folinate is obtained by addition of the dipolar aprotic solvents of claims 3 or 4 or of a mixture of the latter with a polar protic solvent.

6. A process according to claim 5 wherein the polar protic solvent is selected from: methanol, ethanol, n-propanol, isopropanol, n-butanol, sec-butanol, ethylene glycol, 1,2-propylene glycol, 1,3-propylene glycol, formamide, N-methyl-formamide.

7. A process according to anyone of claims 1-6, further comprising the recrystallization of the (6S), respectively (6R) diamino folinate, from a water/aprotic dipolar solvent mixture in a ratio ranging from 1:0.5 (w/w) to 1:20 (w/w) respectively, or from a water/aprotic dipolar solvent/organic protic solvent mixture to give said diastereomer with an optical purity higher than 99%.

8. A process according to claim 1, further comprising the transformation of the (6S), respectively (6R), diamino folinate into the corresponding calcium folinate with an optical purity higher than 99%, said transformation being carried out in water, in the presence of CaCl₂ in a weight ratio CaCl₂/diamine salt ranging from 1 to 6, at a temperature from 0 to 35°C at pH values ranging from 6 to 7.

9. A process according to claim 1, further characterized by recovering from the mother liquors the diamino folinate diastereomer remained in the solution, said recovery comprising:
- dilution of said mother liquors with a dipolar aprotic solvent or with a polar protic solvent, up to precipitation of said diastereomeric salt;
- filtration and recrystallization of the diastereomeric salt from said solvent mixtures in order to give the diamino folinate diastereomer with an optical purity higher than 99%,
or:
- treatment of said mother liquors with a CaCl₂ excess up to a diamine salt/CaCl₂ weight ratio of 1:6 w/w, subsequent dilution with a polar protic solvent, filtration of the precipitated crude calcium salt and final recrystallization from water.

## Patentansprüche

1. Verfahren zur Herstellung, Trennung und Reinigung von (6S) - und (6R) -Diastereomeren von Folinsäuresalzen, umfassend die Hydrolyse von (6RS)-5,10-Methenyl-5,6,7,8-tetrahydrofolsäurechlorid-hydrochlorid und die Trennung der (6R)- und (6S)-Isomeren, dadurch **gekennzeichnet,** daß die Hydrolyse mit einem mindestens dibasischen Amin in Wasser oder in einem Gemisch aus Wasser und aprotischem dipolarem Lösungsmittel durchgeführt wird, und daß die so erhaltenen diastereoisomeren Salze von (6RS)-Folinsäure mit dem mindestens dibasischen Amin durch selektive Kristallisation direkt aus der in der Hydrolysestufe erhaltenen Rohlösung ohne vorherige Reinigung abgetrennt werden, ggf. gefolgt von der Umwandlung in Calciumsalze.

2. Verfahren nach Anspruch 1, dadurch **gekennzeichnet,** daß das mindestens dibasische Amin aus Diaminen der allgemeinen Formeln (I) bis (III) 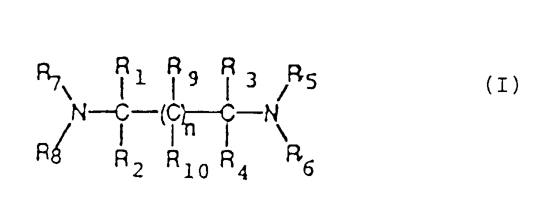 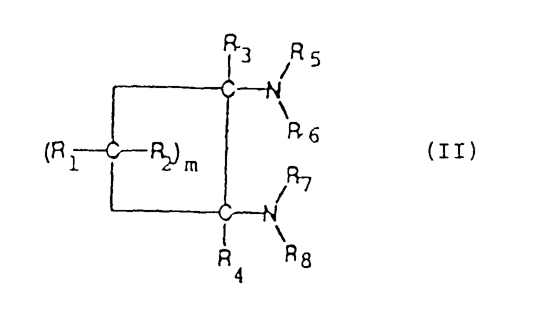 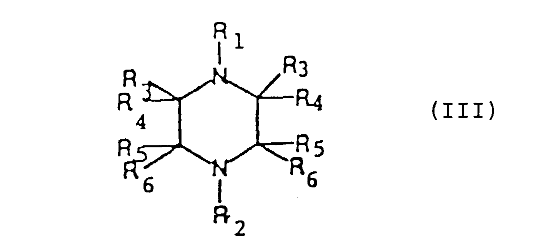 worin:
R₁ bis R₈ die gleich oder verschieden sind, H oder eine lineare oder verzweigte Alkylgruppe, die durch 1 bis 4 OH-Gruppen substituiert ist oder nicht , bedeuten;
R₉ bis R₁₀ die gleich oder verschieden sind, die gleiche Bedeutung wie R₁ bis R₈ haben, oder Hydroxylgruppen bedeuten;
n eine ganze Zahl von 0 bis 6 ist,
m eine ganze Zahl von 2 bis 8 ist,
ausgewählt wird.

3. Verfahren nach Anspruch 2, dadurch **gekennzeichnet,** daß das bevorzugte Amin aus Ethylendiamin, 1,2-Diaminopropan, 1,3-Diaminopropan, 1,3-Diamino-2-hydroxypropran, (cis)-1,2-Diaminocyclohexan, (trans)-1,2-Diaminocyclohexan, Piperazin, 2-Methylpiperazin, 1,4-Dimethylpiperazin, 2,5-Dimethylpiperazin ausgewählt wird.

4. Verfahren nach Anspruch 1, dadurch **gekennzeichnet,** daß das dipolare aprotische Lösungsmittel aus Dimethylformamid, Dimethylacetamid, Dimethylsulfoxid, N-Methylpyrrolidon, Hexamethylphosphoramid ausgewählt wird.

5. Verfahren nach einem der Ansprüche 1 bis 4, dadurch **gekennzeichnet,** daß die Fällung des weniger löslichen Diastereomeren des Diaminofolinats durch Zugabe von dipolaren aprotischen Lösungsmitteln nach Anspruch 3 oder 4 oder eines Gemisches der zuletzt genannten mit einem polaren protischen Lösungsmittel erhalten wird.

6. Verfahren nach Anspruch 5, dadurch **gekennzeichnet,** daß das polare protische Lösungsmittel aus Methanol, Ethanol, n-Propanol, Isopropanol, n-Butanol, sek.-Butanol, Ethylenglykol, 1,2-Propylenglykol, 1,3-Propylenglykol, Formamid, N-Methylformamid ausgewählt wird.

7. Verfahren nach einem der Ansprüche 1 bis 6, dadurch **gekennzeichnet,** daß man weiterhin (6S)- bzw. (6R)-Diaminofolinat aus einem Gemisch aus Wasser und einem aprotischen dipolaren Lösungsmittel in einem Verhältnis von 1:0,5 (Gew./Gew.) bis 1:20 (Gew./Gew.) bzw. aus einem Gemisch aus Wasser/aprotischem dipolarem Lösungsmittel/organischem protischem Lösungsmittel umkristallisiert, wodurch das Diastereomere mit einer optischen Reinheit von mehr als 99% erhalten wird.

8. Verfahren nach Anspruch 1, dadurch **gekennzeichnet,** daß man weiterhin das (6S)- bzw. (6R)-Diaminofolinat in das entsprechende Calciumfolinat mit einer optischen Reinheit von mehr als 99% umwandelt, wobei die Umwandlung in Wasser in Gegenwart von CaCl₂ in einem Gewichtsverhältnis CaCl₂/Diaminsalz im Bereich von 1 bis 6 bei einer Temperatur von 0 bis 35°C bei pH-Werten im Bereich von 6 bis 7 durchgeführt wird.

9. Verfahren nach Anspruch 1, dadurch **gekennzeichnet,** daß man weiterhin die Mutterlaugen des in der Lösung verbliebenen Diaminofolinat-Diastereomeren gewinnt, wobei man
- die Mutterlaugen mit einem dipolaren aprotischen Lösungsmittel oder mit einem polaren protischen Lösungsmittel bis zur Fällung des diastereomeren Salzes verdünnt;
- das diastereomere Salz aus den Lösungsmittelgemischen filtriert und umkristallisiert, wodurch das Diaminofolinat-Diastereomere mit einer optischen Reinheit von mehr als 99% erhalten wird; oder
- die Mutterlaugen mit CaCl₂ in einem Überschuß bis zu einem Gewichtsverhältnis Diaminsalz/CaCl₂ von 1:6 Gew./Gew. behandelt, anschließend mit einem polaren protischen Lösungsmittel verdünnt, das so gefällte rohe Calciumsalz abfiltriert und schließlich aus Wasser umkristallisiert.

## Revendications

1. Un procédé pour la préparation, la séparation et la purification de diastéréoisomères (6S) et (6R) de sels d'acide folinique, comprenant l'hydrolyse de chlorhydrate de chlorure d'acide (6RS)-5,10-méthényl-5,6,7,8-tétrahydrofolique et la séparation des isomères 6R et 6S, caractérisé en ce que l'hydrolyse est effectuée avec une amine au moins dibasique dans de l'eau ou un mélange eau/solvant dipolaire aprotique et en ce que les sels diastéréoisomères de l'acide (6RS) folinique et de l'amine au moins dibasique obtenus sont séparés par cristallisation sélective directement à partir de la solution brute obtenue par l'étape d'hydrolyse, sans purification préalable, suivie facultativement par la conversion en sels de calcium.

2. Un procédé selon la revendication 1, dans lequel l'amine au moins dibasique est choisie parmi les diamines des formules générales (I) à (III) 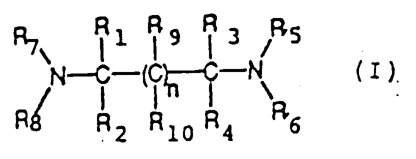 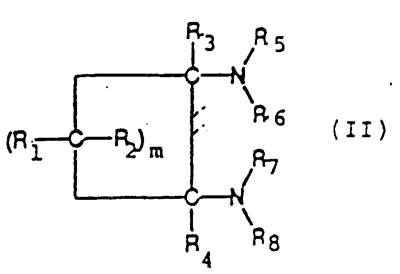 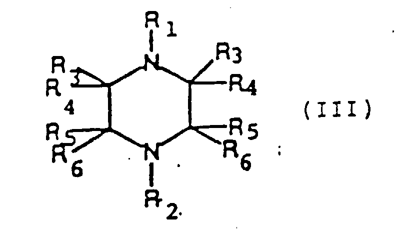 où :
R₁ à R₈ qui sont identiques ou différents, sont H ou un groupe alkyle linéaire ou ramifié, substitué ou non par 1 à 4 groupes OH ;
R₉ à R₁₀, qui sont identiques ou différents, ont les mêmes significations que R₁-R₈ ou représentent des groupes hydroxyle ;
n est un nombre entier de 0 à 6,
m est un nombre entier de 2 à 8.

3. Un procédé selon la revendication 2, dans lequel l'amine préférée est choisie parmi : l'éthylènediamine, le 1,2-diaminopropane, le 1,3-diaminopropane, le 1,3-diamino-2-hydroxypropane,le (*cis*)-1,2-diaminocyclohexane, le *(trans)*-1,2-diaminocyclohexane, la pipérazine, la 2-méthylpipérazine, la 1,4-diméthylpipérazine, la 2,5-diméthylpipérazine.

4. Un procédé selon la revendication 1, dans lequel le solvant aprotique dipolaire est choisi parmi : le diméthylformamide, le diméthylacétamide, le diméthylsulfoxyde, la *N*-méthylpyrrolidone, l'hexaméthylphosphoramide.

5. Un procédé selon l'une quelconque des revendications 1 à 4, dans lequel la précipitation du diastéréoisomère le moins soluble dudit diamino-folinate est réalisée par addition des solvants aprotiques dipolaires des revendications 3 ou 4 ou d'un mélange de ceux-ci avec un solvant protique polaire organique.

6. Un procédé selon la revendication 5, dans lequel le solvant protique polaire organique est choisi parmi : le méthanol, l'éthanol, le *n*-propanol, l'isopropanol, le *n*-butanol, le *sec*-butanol, l'éthylène-glycol, le 1,2-propylène-glycol, le 1,3-propylène-glycol, le formamide, le *N*-méthylformamide.

7. Un procédé selon l'une quelconque des revendications 1 à 6, comprenant de plus la recristallisation du (6S), respectivement (6R), diaminofolinate dans un mélange eau/solvant aprotique dipolaire en un rapport compris entre 1:0,5 (en poids) et 1:20 (en poids), respectivement, ou dans un mélange eau/solvant aprotique dipolaire/solvant protique organique pour donner ledit diastéréoisomère avec une pureté optique supérieure à 99 %.

8. Un procédé selon la revendication 1, comprenant de plus la transformation du (6S), respectivement (6R), diamino-folinate en le folinate de calcium correspondant ayant une pureté optique supérieure à 99 %, ladite transformation étant effectuée dans l'eau, en présence de CaCl₂ en un rapport en poids CaCl₂/sel de diamine compris entre 1 et 6, à une température de 0 à 35°C à des valeurs de pH comprises entre 6 et 7.

9. Un procédé selon la revendication 1, caractérisé de plus par la récupération, à partir des liqueurs-mères, du diastéréoisomère de diamino-folinate resté dans la solution, ladite récupération comprenant :
- une dilution desdites liqueurs-mères avec un solvant aprotique dipolaire ou avec un solvant protique polaire, jusqu'à précipitation dudit sel diastéréoisomère ;
- une filtration et une recristallisation du sel diastéréoisomère dans lesdits mélanges de solvants afin d'obtenir le diastéréoisomère de diamino-folinate avec une pureté optique supérieure à 99 %,
ou :
- un traitement desdites liqueurs-mères avec un excès de CaCl₂ jusqu'à un rapport sel de diamine/CaCl₂ de 1:6 en poids, une dilution subséquente avec un solvant protique polaire, une séparation par filtration du sel de calcium brut précipité et une recristallisation finale dans l'eau.
